# EUROPEAN PATENT SPECIFICATION

(11) **EP 2 481 414 B1**
(45) Date of publication and mention of the grant of the patent: **31.07.2013**
(21) Application number: 12150915.2
(22) Date of filing: 12.01.2012
(51) Int. Cl.: A61K 31/785, A61K 31/795, C08F 20/52, C08F 26/02, C08K 5/151, C08L 39/00

(54) **A process for the preparation of cross-linked polyallylamines or pharmaceutically acceptable salts thereof**
Verfahren zur Herstellung von vernetzten Polyallylaminen oder ihrer pharmazeutisch akzeptablen Salze
Procédé de préparation de polyallylamines réticulés ou sels acceptables pharmaceutiques correspondants

(30) Priority: 01.02.2011 IT MI20110126
(43) Date of publication of application: 01.08.2012
(73) Proprietor: Chemi SPA, 20092 Cinisello Balsamo (MI) (IT)
(72) Inventor: Gaboardi, Mauro, 28100 Novara (IT); Oldani, Erminio, 20010 Cornaredo (MI) (IT)
(74) Representative: Pistolesi, Roberto

(56) References cited:
- EP-A1- 0 716 606
- WO-A1-01/05408

## Description

The present invention relates to a process for the preparation of cross-linked polyallylamines or pharmaceutically acceptable salts thereof. The invention also relates to a process for the preparation of Sevelamer, or pharmaceutically acceptable salts thereof, preferably hydrochloride or carbonate salts, used in the treatment of hyperphosphatemia, and having good flowability, low porosity and/or a swelling index of between 7 and 9.

### FIELD OF THE INVENTION

Hyperphosphatemia often accompanies diseases connected with renal insufficiency which is an ever more widespread chronic condition associated with a progressive loss of kidney function which often complicates the course of other diseases such as diabetes and cardiovascular diseases. Particularly when it is present for long periods of time, hyperphosphatemia leads to serious anomalies in the metabolism of phosphorus and calcium, causing calcification of the soft tissues, for example, joints, lungs, eyes, vessels, arteries, etc.

Hyperphosphatemia is generally treated with phosphate group binders such as anion-exchange polymers. These polymers lead to a reduction in the blood phosphate level without increasing the absorption of other clinically undesirable compounds. Cross-linked polyallylamines, Sevelamer for example, are amongst the anion-exchange polymers that are used widely in the treatment of hyperphosphatemia. Sevelamer, which is polyallylamine cross-linked with epichlorohydrin, was marketed initially as the hydrochloride and more recently, as the carbonate. Sevelamer carbonate has similar efficacy to Sevelamer hydrochloride but is better tolerated by patients.

Sevelamer is a compound of structural formula (I), 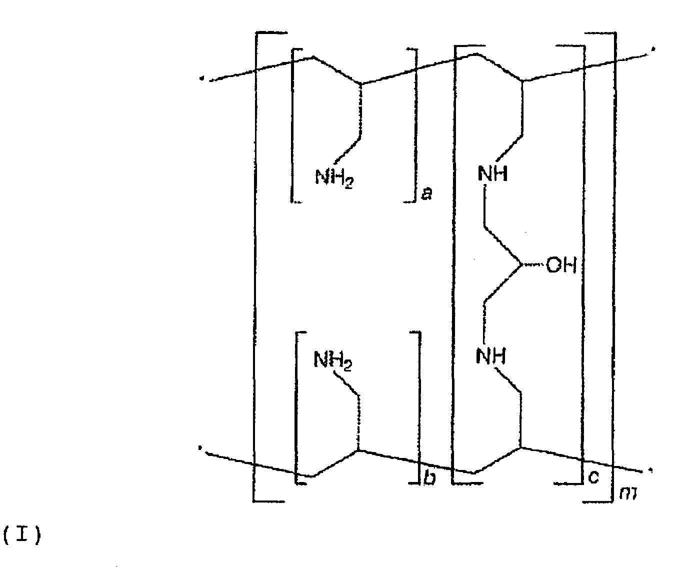 and empirical formula (Ia):

[ (C₃H₇N) _{a+b} · (C₉H₁₇N₂O)_{c}]ₘ

in which:
- a+b : c = 9 : 1;
- m is a whole number.

It is described in European patent EP716606B1 and marketed under the name Renvela^{®} (Sevelamer carbonate) and Renagel^{®} (Sevelamer hydrochloride).

Few documents describe the preparation of Sevelamer. European patent EP716606B1 describes the preparation of Sevelamer hydrochloride by the polymerization of allylamine in the presence of an initiator, and subsequent cross-linking with epichlorohydrin.

European patent EP1175451B1 describes the preparation of Sevelamer by the reaction of polyallylamine and a cross-linking agent such as epichlorohydrin or 1,3-dichloro-2-propanol, in a basic environment, in water and in a water-miscible solvent.

Patent application WO2009/010531 describes the preparation of Sevelamer by the reaction of allylamine with 1,3-bis-allylamino-2-propanol as cross-linking agent.

Patent application WO2009/125433 describes the preparation of Sevelamer carbonate by cross-linking of polyallylamine carbonate with epichlorohydrin, optionally in the presence of an emulsifier and/or surfactant. Polyallylamine carbonate is obtained by basic treatment of polyallylamine hydrochloride and subsequent reaction with a carbonate source.

Patent application WO2001/18073 describes the preparation of Sevelamer with low cohesiveness which is achieved only after several steps which provide for the reaction of an aqueous polyallylamine solution with a cross-linking agent such as epichlorohydrin, washing of the aqueous solution with an alcohol/water solution in the presence of a surfactant, drying of the cross-linked polymer, grinding, and finally sieving.

Patent application WO 01/05408 describes the reaction of polyallylamines with "modifying agents" for the preparation of polyallylamine derivatives in which the nitrogen atoms of the amine groups are bound to hydrophobic substituents of the modifying agents. In particular, WO 01/05408 describes the use of glycidol as modifying agent. The reaction of the polyallylamine with glycidol does not, however, enable it to be cross-linked, as shown in Comparative Example 6 of the present application.

There is therefore still a need to provide a process for the preparation of cross-linked polyallylamines or pharmaceutically acceptable salts thereof which is repeatable, can easily be industrialized, without the use of toxic reagents, and which permits the direct production of cross-linked polyallylamines or pharmaceutically acceptable salts thereof with good flowability and/or low porosity, without further process steps such as, for example, grinding, lyophilization, or spray-drying.

### SUMMARY OF THE INVENTION

It has now surprisingly been found that cross-linked polyallylamines or pharmaceutically acceptable salts thereof can be produced by a simple process that can be used at an industrial level to produce large quantities of product without the use of toxic reagents and without further process steps.

The present invention thus relates to a process for the preparation of cross-linked polyallylamines or pharmaceutically acceptable salts thereof which comprises the following steps:
a) polymerization of allylamine of formula (II) 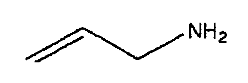 in the presence of a radical initiator, in an acid environment, in a polar solvent or a mixture of polar solvents to give a polyallylamine;
b) cross-linking of the polyallylamine obtained in step a) in a basic environment with a cross-linking agent of formula (III) 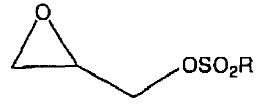 in which R is a C₁-C₆ alkyl group, or an aryl group optionally substituted with one or more alkyl groups and/or electron-attractor groups, in a polar solvent or a mixture of polar solvents to give a polymer;
c) salification of the polymer obtained in step b), preferably with a pharmaceutically acceptable salt; and
d) optional conversion of the salified polymer obtained in step c) into another pharmaceutically acceptable salt.

A further aspect of the present invention relates to the preparation of Sevelamer, or pharmaceutically acceptable salts thereof, preferably hydrochloride or carbonate salts. Finally, a subject of the present invention is Sevelamer or pharmaceutically acceptable salts thereof, preferably hydrochloride or carbonate salts, obtainable by the above-mentioned process and, in particular, Sevelamer or pharmaceutically acceptable salts thereof, preferably hydrochloride or carbonate salts, having an angle of rest of between 25.0° and 27.0° and/or a true density of between 1.25 and 1.32 and/or a swelling index of between 7 and 9.

### DETAILED DESCRIPTION OF THE INVENTION

All of the terms that are used in the present application should be understood in their conventional meaning as they are known in the art unless indicated to the contrary. Other more specific definitions for some terms as used in this application are given below and always apply throughout the description and the claims unless a different definition explicitly gives a wider definition.

The term "polymer" refers not only to a homopolymer but also to a copolymer.

The term "polyallylamine" refers to a polymer which comprises allylamine as a repetitive unit.

The term "repetitive unit" refers to a part of the polymer chain which is derived from a single molecule of a monomer. The term "cross-linked" refers to interconnections between the polymer chains.

The term "cross-linking agent" refers to an agent which brings about cross-linking, branching, or a combination thereof.

The term "C₁-C₆ alkyl" refers to a branched or linear hydrocarbon containing from 1 to 6 carbon atoms. Examples of C₁-C₆ alkyl groups include but are not limited to methyl, ethyl, *n*-propyl, isopropyl, *n*-butyl, *sec-*butyl, isobutyl, *tert*-butyl, *n*-pentyl, *n*-hexyl. The C₁-C₆ alkyl may optionally be substituted with one or more electron-attractor groups. The term "aryl" refers to an aromatic carbo-cycle constituted by 1 or 2 rings fused or bound together by a single bond. Examples of aryl groups include but are not limited to phenyl, and α- or β-naphthyl. The aryl group may optionally be substituted with one or more C₁-C₆ alkyl groups e/o electron-attractor groups.

The term "electron-attractor group" refers to a group which tends to stabilize carbanions by helping to disperse their negative charge. Examples of electron-attractor groups include but are not limited to, halogens, such as fluoro, chloro, bromo, iodo groups; the nitro group (NO₂); and the nitrile group (CN).

The term "pharmaceutically acceptable salts" refers to nontoxic inorganic or organic salts. Examples of pharmaceutically acceptable salts include but are not limited to: carbonate, hydrochloride, hydrobromide, sulphate, hydrogen sulphate, citrate, maleate, fumarate, trifluoroacetate, 2-naphthalenesulphonate, and paratoluenesulphonate. Further information on pharmaceutically acceptable salts can be found in pharmaceutical chemistry manuals, for example: Handbook of pharmaceutical salts, P. Stahl, C. Wermuth, WILEY-VCH (Eds., 2008, pp. 127-133).

The term "one pot" refers to two or more consecutive reactions which are carried out without isolating the respective intermediate product or products.

The term "approximately" includes the range of the experimental error which may occur in a measurement.

The present invention relates to a process for the preparation of cross-linked polyallylamines or pharmaceutically acceptable salts thereof which comprises the following steps:
a) polymerization of allylamine of formula (II) 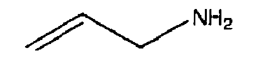 in the presence of a radical initiator, in an acid environment, in a polar solvent or a mixture of polar solvents to give a polyallylamine;
b) cross-linking of the polyallylamine obtained in step a) in a basic environment with a cross-linking agent of formula (III) 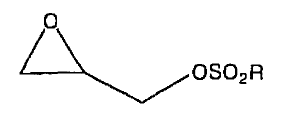 in which R is a C₁-C₆ alkyl group, or an aryl group optionally substituted with one or more alkyl groups and/or electron-attractor groups, in a polar solvent or a mixture of polar solvents to give a polymer;
c) salification of the polymer obtained in step b), preferably with a pharmaceutically acceptable salt; and,
d) optional conversion of the salified polymer into another pharmaceutically acceptable salt.

A further aspect of the present invention relates to the preparation of Sevelamer, or pharmaceutically acceptable salts thereof, preferably hydrochloride or carbonate salts. Another aspect of the present invention relates to Sevelamer or pharmaceutically acceptable salts thereof, preferably hydrochloride or carbonate salts, obtainable by the process of the present invention and preferably having an angle of rest of between 25.0° and 27.0° and/or a true density of between 1.25 and 1.32 and/or a swelling index of between 7 and 9.

Preferably, at least one and, even more preferably, all of steps a), b), c) and d) of the process of the present invention are carried out in "one pot".

The allylamine of formula (II) is polymerized in the presence of a radical initiator in an acid environment in a polar solvent or a mixture of polar solvents.

Any radical initiator known to persons skilled in the art may be used in the present invention. The radical initiator is preferably selected from nitrogenous compounds, more preferably azobisisobutyronitrile, 2,2'-azobis(2-amidinopropane) dihydrochloride, or 2,2'-azobis[2-(2-imidazolin-2-yl)propane] dihydrochloride (VA-044); organic peroxides, more preferably benzoyl peroxide, di-tert-amyl peroxide, dicumyl peroxide, isopropylbenzene peroxide; inorganic peroxides, more preferably hydrogen peroxide, sodium peroxide, or potassium peroxide; compounds that can be activated by thermal or redox reactions, or a combination thereof; more preferably, the radical initiator is a nitrogenous compound, even more preferably 2,2'-azobis(2-amidinopropane) dihydrochloride o 2,2'-azobis[2-(2-imidazolin-2-yl)propane] dihydrochloride (VA-044).

The molar ratio of allylamine to radical initiator is preferably between 20:1 and 50:1, more preferably between 20:1 and 35:1.

The polymerization reaction of step a) is performed in the presence of an acid. The acid is preferably an inorganic acid, more preferably hydrochloric acid, hydrobromic acid, sulphuric acid, nitric acid, phosphoric acid, or the like. Even more preferably, it is hydrochloric acid.

Any polar solvent known to persons skilled in the art may be used in the process of the present invention. The polar solvent is preferably selected from water; an ester, more preferably ethyl acetate or butyl acetate; a nitrile, more preferably acetonitrile; an ether, more preferably tetrahydrofuran or dioxane; an amide, more preferably dimethylformamide or dimethylacetamide; a sulphoxide, more preferably dimethylsulphoxide, or mixtures thereof, even more preferably water.

The polymerization reaction is performed at a temperature such that the reaction goes to completion, preferably at a temperature between the decomposition temperature of the radical initiator and the reflux temperature of the reaction mixture, more preferably between 60 and 80°C.

The polyallylamine thus obtained can be isolated by separation techniques well known to persons skilled in the art, such as: extraction, filtration, crystallization, precipitation, and the like, or it may be used directly, without isolation or purification, in the subsequent cross-linking step b). The polyallylamine is preferably not isolated.

After the pH of the polymerization mixture of step a) has been brought to basic values by the addition of a base, the polyallylamine is reacted with the cross-linking agent of formula (III) 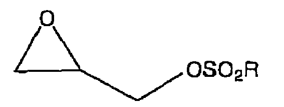 in which R is a C₁-C₆ alkyl group, or an aryl group optionally substituted with one or more alkyl groups and/or electron-attractor groups. Preferably, R is selected from methyl, trifluoromethyl, *p*-tolyl, and *m*-nitro-phenyl; more preferably *m*-nitro-phenyl or *p*-tolyl. The cross-linking agent of formula (III) is preferably *m*-nitrobenzensulphonyl glycidol or tosyl glycidol.

The cross-linking reaction of step b) is performed in the presence of a base. Any base known to persons skilled in the art may be used in the present invention to bring the pH of the solution to basic values. Preferably, the base is an inorganic base, more preferably sodium or potassium hydroxide or sodium or potassium carbonate, even more preferably sodium hydroxide.

The cross-linking agent of formula (III), in solid form or in solution, is added to the polyallylamine solution. Any polar solvent or mixture of polar solvents known to persons skilled in the art may be used in the present invention. Preferably, the compound of formula (III) is used in solution in a polar solvent, more preferably water, even more preferably ethyl acetate.

The concentration of the cross-linking agent of formula (III) is preferably between 0.5 and 2 mol/l, more preferably between 1.2 and 1.8 mol/l.

The cross-linking agent of formula (III) may be prepared by any method known to persons skilled in the art, for example, as described in J. Org. Chem., 1989, 54, 1295-1304. For example, the compound of formula (III) may be prepared as shown by way of example in Scheme 1, by reacting the glycidol of formula (IV), which is a commercial product, with the sulphonyl halide R-SO₂X of formula (V) in which R has the meaning defined above and X is a halogen, for example, chlorine, bromine, or iodine. The reaction takes place in a basic environment, preferably with the use of an organic base such as an amine, for example, triethylamine or diisopropylethylamine; an amidine, for example, 1,8-diazadicyclo[5,4,0]undec-7-ene; or an aromatic base, for example, pyridine. More preferably, the base is triethylamine or diisopropylethylamine.

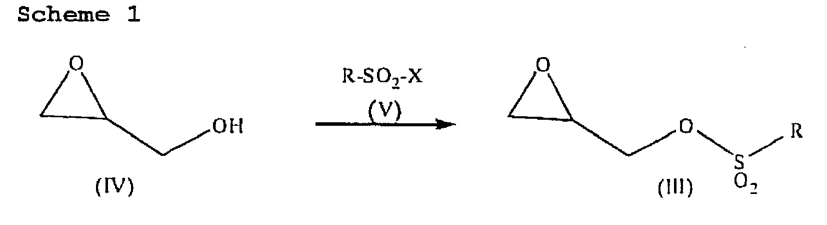

The compound of formula (III) may be isolated in accordance with techniques well known to persons skilled in the art such as, for example, extraction, filtration, crystallization, precipitation, and the like, or may be used without further separation and purification, by adding the solution containing it directly to the polyallylamine mixture.

After the addition of the cross-linking agent of formula (III), the reaction mixture is preferably stirred for 6-14 hours, more preferably 10-12 hours. An easily handled, solid polymer is obtained. The reaction mixture is then brought to acid pH by the addition of an acid to give the salified polymer.

Any acid known to persons skilled in the art may be used in the present invention to salify the polymer. Preferably, the acid is a pharmaceutically acceptable acid, more preferably an inorganic acid, even more preferably hydrochloric acid. The salified polymer can be isolated by techniques known to persons skilled in the art, such as precipitation, filtration, with or without pressure and/or under vacuum, crystallization, centrifuging, decantation, and the like. Preferably, the salified polymer is a hydrochloride or a carbonate, more preferably a carbonate.

Any method known to persons skilled in the art may be used to obtain the polymer carbonate. Preferably, the polymer hydrochloride is converted into the corresponding carbonate by reaction with a carbonate source. Preferably, according to an aspect of the invention, the polymer hydrochloride is suspended in water and a carbonate source, for example, sodium or potassium carbonate, preferably sodium carbonate, is added. The reaction mixture is brought to a suitable temperature between room temperature and the reflux temperature of the reaction mixture, preferably between room temperature and 40°C. The polymer in the form of the carbonate salt is separated from the mother liquor, washed and dehydrated.

The process of the present invention permits the preparation of cross-linked polyallylamines or pharmaceutically acceptable salts thereof, preferably Sevelamer or pharmaceutically acceptable salts thereof, more preferably hydrochloride or carbonate salts, even more preferably the carbonate salt.

The process of the present invention is an efficient, inexpensive process with good yields, is repeatable, and takes place in very mild conditions without the use of surfactants and/or toxic substances. The process of the present invention can easily be used on an industrial scale to prepare large quantities of product and enables the final product to be produced with well-defined chemical and physical characteristics which lead to easy handling of the solid obtained, without the need for further processes such as grinding, lyophilization, or spray-drying.

In the pharmaceutical field, the flowability of the powder and the true density of the product are factors which are extremely important for ease of handling of the solid, for optimal storage, and for the preparation of pharmaceutical formulations.

Sevelamer and the pharmaceutically acceptable salts thereof such as the hydrochloride and carbonate salts which can be obtained by the process of the present invention have good flowability, good porosity, and/or a swelling index of between 7 and 9.

The flowability of a powder is determined by measurement of the corresponding angle of rest. The angle of rest measured on Sevelamer or the pharmaceutically acceptable salts thereof that are obtained by the process described in the present invention is between 25.0° and 27.0°.

The angle of rest was measured in accordance with the method described in European Pharmacopoeia 6.0 (2010); 100 g of product were introduced into a hopper having a blocked hole placed 10cm from a surface. The hole was opened, the powder was allowed to flow out, and the flow time was measured.

The porosity of a powder is determined by measurement of its true density. A high true density indicates low porosity, given that there are fewer empty interstitial spaces. Sevelamer and the pharmaceutically acceptable salts thereof such as the hydrochloride and carbonate salts which can be obtained by the process described in the present invention have a true density of between 1.25 and 1.32.

The true density was measured in accordance with the helium pycnometry method as described in the ASTM B923-02 "*Standard Test Method for Metal Powder Skeletal Density by Helium or Nitrogen Pycnometry".*

Sevelamer and the pharmaceutically acceptable salts thereof such as the hydrochloride and carbonate salts which can be obtained by the process described in the present invention have a swelling index of between 7 and 9.

A high swelling index means that the polymer incorporates water until it adopts a volume equal to the value of the index. The swelling index was measured in accordance with the procedure described in patent WO 01/18073 which is incorporated herein by reference.

The phosphate sequestering power of Sevelamer and the pharmaceutically acceptable salts thereof such as the hydrochloride and carbonate salts which can be obtained by the process described in the present invention is between 5.0 and 6.0 mmol/g.

In a preferred embodiment of the present invention, in order to obtain Sevelamer or the pharmaceutically acceptable salts thereof, the allylamine polymerization reaction is performed with the use, as radical initiator, of 2,2'-azobis[2-(2-imidazolin-2-yl)propane] dihydrochloride (VA-044) in a molar ratio of allylamine to radical initiator of approximately 23 : 1 and hydrochloric acid as the acid. The polar solvent used is water. The polymerization reaction is performed at a temperature of 30-50° C, preferably approximately 40°C.

The reaction for the cross-linking of the polyallylamine thus obtained is performed in a basic environment by the addition of sodium hydroxide and one of the cross-linking agents of formula (IIIa) or (IIIb) given below, that is, tosyl glycidol or m-nitrobenzensulphonyl glycidol, respectively, in ethyl acetate.

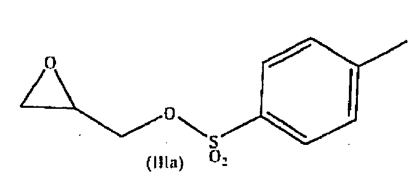

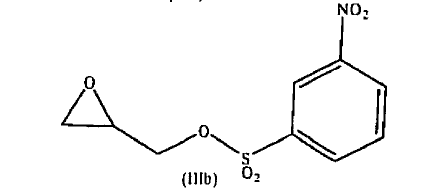

The reaction takes approximately 10-12 hours.

The pH is then brought to acidity by the addition of hydrochloric acid. The solid thus obtained, which corresponds to Sevelamer hydrochloride, is suspended in water and sodium carbonate is added. The temperature is brought to approximately 30-35°C. After washing with isopropanol, Sevelamer carbonate is obtained.

Even though characteristic aspects of the present invention have been described, modifications and equivalents that are clear to persons skilled in the art are included in the present invention.

The present invention will be illustrated below by means of some examples which should not be seen as limiting of the scope of the invention.

### EXAMPLES

The following abbreviations refer to the definitions given below, respectively:
AcOEt (ethyl acetate); TEA (triethylamine); TLC (Thin Layer Chromatography); HCl (hydrochloric acid); h (hour); r.t. (room temperature); VA-044 (2,2'-azobis[2-(2-imidazolin-Z-yl)propane] dihydrochloride).

### Example 1

### Synthesis of tosyl glycidol. (IIIa)

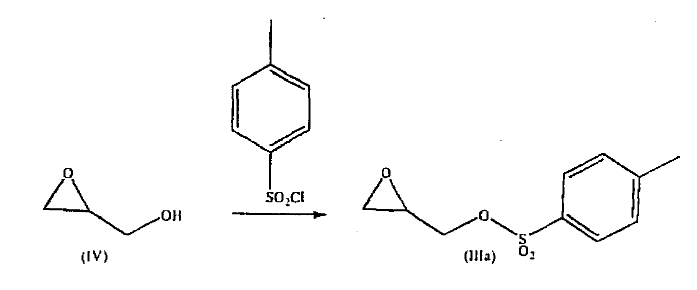

AcOEt (800 ml) and TEA (162.72 g, 1.61 mol) were introduced into a 2 litre, 3-necked flask with a magnetic stirrer, thermometer, and cooler and tosyl chloride (286 g, 1.5 mol) was added in portions. The internal temperature was brought to 0°C and glycidol (IV) was added dropwise over a period of 1h. The mixture was left to react overnight and was allowed to return slowly to r.t. The reaction was monitored by TLC (hexane/AcOEt 8:2). Upon completion of the reaction, 250 ml of H₂O were added and two clear phases were obtained. The phases were separated and the organic phase was washed with 100 ml of H₂O containing approximately 10 g of NaCl. Dehydration was performed with sodium sulphate and the AcOEt was evaporated. The pure tosyl glycidol (IIIa) was obtained as a brilliant white, low melting, waxy solid (370 g). Yield: 96%.
¹H-NMR (CDCl₃, 200MHz ) : 7.791 (d 2H); 7.344 (d 2H); 4.23 (dd 1H); 3.937 (dd 1H); 3.157 (m 1H); 2.798 (m 1H); 2.575 (m 1H); 2.439 (s 3H).

### Example 2

### Synthesis of m-nitrobenzenesulphonyl glycidol (IIIb)

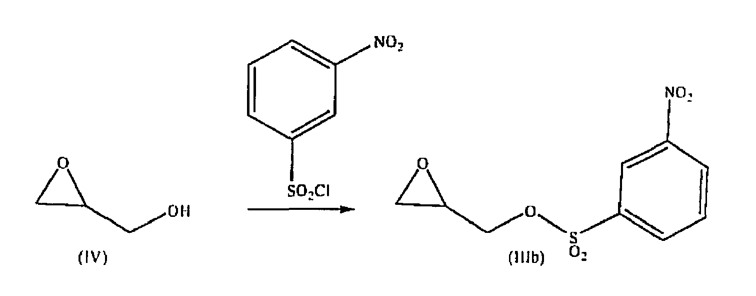

AcOEt (20 ml) and TEA (2.53 g, 0.025 mol) were introduced into a 50 ml, 3-necked flask with a magnetic stirrer, thermometer, and cooler and *m*-nitrobenzenesulphonyl chloride (5 g, 0.0226 mol) was added in portions. The internal temperature was brought to 0°C and glycidol (IV) (1.84 g, 0.025 mol) was added dropwise over a period of 1h. The mixture was left to react overnight and was allowed to return slowly to r.t. The reaction was monitored by TLC (hexane/AcOEt 8:2). Upon completion of the reaction, 10 ml of H₂O was added and two clear phases were obtained. The phases were separated and the organic phase was washed with 10 ml of H₂O containing approximately 1 g of NaCl. Dehydration was performed with sodium sulphate and the AcOEt was evaporated. Pure m-nitrobenzenesulphonyl glycidol (IIIb) was obtained as a brownish-yellow, low melting, waxy solid (4.6 g). Yield: 78.6%.
¹H-NMR (CDCl₃, 300MHz): 8.75 (d 1H); 8.52 (dd, 1H); 8.24 (dd 1H); 7.81 (t 1H); 4.45 (dd 1H); 4.03 (dd 1H); 3.20 (m 1H); 2.83 (m 1H); 2.61 (m 1H).

### Example 3

### Preparation of Sevelamer, hydrochloride with the use of tosyl glycidol (IIIa) as cross-linker

37% HCl (38 g, 0.385 mol) was introduced into a 250 ml, 4-necked flask with a mechanical stirrer, cooler, and thermometer and diluted with water (24 ml). The temperature was brought to 2-4°C and allylamine (20 g, 0.35 mol) was added dropwise. Upon completion of the dropwise addition, the temperature was brought to 72-73°C. During the heating, at approximately 40°C, the VA-044 initiator (4.8 g, 0.015 mol) was added. The mixture was left to react for 4h. The aqueous solution was clear and the viscosity increased over time. The solution was brought to pH 10 with 50% NaOH and the solution of tosyl glycidol (IIIa) in AcOEt (20 g, 0.0877 mol in 50 ml) was added dropwise. The addition was performed over a period of 1 hour. The mixture was left to react overnight and a gelatinized product was obtained. Further AcOEt (80 ml) was added. The mixture was brought to acid pH with 37% HCl and filtered through cloth. The solid was washed with a water/isopropanol mixture (2 x 250 ml) and with isopropanol (2 x 250 ml). 50 g of a moist solid was obtained. The solid was dried in an oven to give 38 g of still moist Sevelamer hydrochloride which was used unchanged in the next step.

### Example 4

### Preparation of Sevelamer carbonate

Sevelamer hydrochloride (28 g) was suspended in water (420 ml). Sodium carbonate (17.5 g, 0.165 mol) was added during stirring. The temperature was brought to 30-35°C and stirring was continued for approximately 30 minutes. 200 ml of isopropanol was added and the mixture was filtered in a Buchner filter. The solid was washed with a (1:1) water/isopropanol mixture (2 x 250 ml) and with isopropanol (250 ml). 35 g of a moist solid was obtained. The product was dried in an oven under vacuum giving 16 g of Sevelamer carbonate.

### Example 5

### Preparation of Sevelamer with the use of m-nitrobenzenesulphonyl glycidol (IIIb) as cross-liner

37% HCl (4.8 g, 0.0487 mol) was introduced into a 3-necked, 50 ml flask with a magnetic stirrer, cooler, and thermometer and was diluted with H₂O (2 ml). The temperature was brought to 2-4°C and allylamine (2.53 g, 0.0443 mol) was added dropwise. Upon completion of the dropwise addition, the temperature was brought to 72-73°C. During the heating, at approximately 40°C, the VA-044 initiator (0.62 g, 0.0019 mol) was added. The mixture was left to react for 4h. The aqueous solution was clear and the viscosity increased over time. When the 4 h had elapsed, the excess water (approximately 15 ml) was distilled, the solution was brought to pH 10 with 50% NaOH (2 g) and the solution of m-nitrobenzenesulphonyl glycidol (IIIb) in AcOEt (4.3 g, 0.0166 mol in 11 ml) was added dropwise. The addition was performed over a period of approximately 1 hour. The mixture was left to react for 14 hours at 70°C. Gelatinization was observed after only half an hour. A further 80 ml of AcOEt was added to improve the breaking-up of the product. The mixture was brought to acid pH with 37% HCl and filtered through cloth. The solid was washed with a water/isopropanol mixture (3:1) (2 x 50 ml) and with isopropanol (3 x 50 ml). The product was dried in an oven at 60°C to give 6.68 g of Sevelamer hydrochloride.

### Example 6 (comparative)

### Preparation of Sevelamer, hydrochloride, with the use of glycidol as cross-liner

37% HCl (3.49 g, 0.0957 mol) was introduced into a 4-necked, 100 ml flask with a mechanical stirrer, cooler, and thermometer and diluted with water (6 ml). The temperature was brought to 2-4°C and allylamine (4.97 g, 0.0870 mol) was added dropwise. Upon completion of the dropwise addition, the temperature was brought to 72-73°C. During the heating, at approximately 40°C, the VA-044 initiator (1.21 g, 0.00373 mol) was added. The mixture was left to react for 4 h. The aqueous solution was clear and the viscosity increased over time. The solution was brought to pH 10 with 50% NaOH and the solution of glycidol in AcOEt (1.61 g, 0.0218 mol in 12.5 ml) was added dropwise. The addition was carried out over a period of 1 hour. The mixture was left to react overnight and the formation of Sevelamer was not noted since an emulsion was obtained which, upon the addition of water, gave rise to a clear, and hence polymer-free, solution.

## Claims

1. A process for the preparation of cross-linked polyallylamines or pharmaceutically acceptable salts thereof, which comprises the following steps:
a) polymerization of allylamine of formula (II) 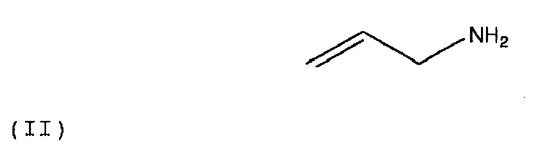 in the presence of a radical initiator, in an acid environment, in a polar solvent or a mixture of polar solvents to give a polyallylamine;
b) cross-linking of the polyallylamine obtained in step a) in a basic environment with a cross-linking agent of formula (III) 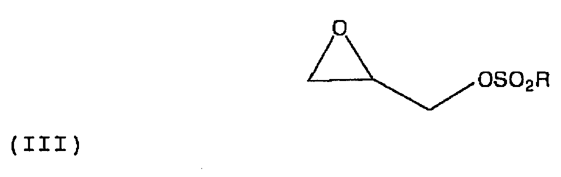 in which R is a C₁-C₆ alkyl group, or an aryl group optionally substituted with one or more alkyl groups and/or electron-attractor groups, in a polar solvent or a mixture of polar solvents to give a polymer;
c) salification of the polymer obtained in step b), preferably with a pharmaceutically acceptable salt; and,
d) optional conversion of the salified polymer into another pharmaceutically acceptable salt.

2. A process according to Claim 1, **characterized in that** the radical initiator is selected from nitrated compounds, preferably azobisisobutyronitrile, 2,2'-azobis(2-amidinopropane) dihydrochloride, or 2,2'-azobis[2-(2-imidazolin-2-yl)propane] dihydrochloride, organic peroxides, preferably benzoyl peroxide, di-*tert*-amyl peroxide, dicumyl peroxide, isopropylbenzene peroxide, inorganic peroxides, preferably hydrogen peroxide, sodium peroxide, potassium peroxide, compounds that can be activated by thermal or redox reactions, or a combination thereof, more preferably, the radical initiator is a nitrated compound, even more preferably 2,2'-azobis(2-amidinopropane) dihydrochloride or 2,2'-azobis[2-(2-imidazalin-2-yl)propane] dihydrochloride.

3. A process according to Claim 1, **characterized in that** the molar ratio of allylamine to radical initiator is between 20:1 and 50:1, preferably between 20:1 and 35:1.

4. A process according to Claim 1, **characterized in that** the polymerization reaction of step a) is performed in the presence of an inorganic acid, preferably hydrochloric acid, hydrobromic acid, sulphuric acid, nitric acid, or phosphoric acid, more preferably hydrochloric acid.

5. A process according to Claim 1, **characterized in that** the polar solvent is selected from water, an ester, preferably ethyl acetate or butyl acetate, a nitrile, preferably acetonitrile, an ether, preferably tetrahydrofuran or dioxane, an amide, preferably dimethylformamide or dimethylacetamide, a sulphoxide, preferably dimethylsulphoxide, or mixtures thereof, more preferably water.

6. A process according to Claim 1, **characterized in that** the polymerization temperature of step a) is between the decomposition temperature of the radical initiator and the reflux temperature of the reaction mixture, preferably between 60 and 80°C.

7. A process according to Claim 1, **characterized in that** the R group of the cross-linking agent of formula (III) of step b) 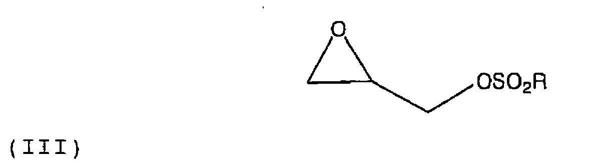 is selected from methyl, trifluoromethyl, *p*-tolyl, and *m-*nitro-phenyl, preferably *m*-nitro-phenyl or *p*-tolyl.

8. A process according to Claim 7, **characterized in that** the cross-linking agent of formula (III) is *m-*nitrobenzenesulphonyl glycidol or tosyl glycidol.

9. A process according to Claim 1, **characterized in that** the cross-linking agent of formula (III) is added to the polyamine of step a) in solid form or in solution, preferably in solution in a polar solvent, more preferably in solution in water or ethyl acetate.

10. A process according to Claim 1, **characterized in that** the concentration of the cross-linking agent of formula (III) in solution is between 0.5 and 2 mol/l, preferably between 1.2 and 1.8 mol/l.

11. A process according to Claim 1 **characterized in that** the cross-linking reaction of step b) is performed in the presence of a base, preferably an inorganic base, more preferably sodium or potassium hydroxide, or sodium or potassium carbonate, even more preferably sodium hydroxide.

12. A process according to Claim 1, **characterized in that** the salification of step c) takes place by treatment with an acid, preferably a pharmaceutically acceptable acid, more preferably an inorganic acid, even more preferably hydrochloric acid.

13. A process according to Claim 1, **characterized in that** the pharmaceutically acceptable salt of step d) is carbonate.

14. A process according to Claim 1, **characterized in that** at least one, and even more preferably all, of steps a), b), c) and d) of the process of the present invention are performed in "one pot".

15. A process according to any one of the preceding claims, **characterized in that** the cross-linked polyallylamine is Sevelamer and that the pharmaceutically acceptable salts thereof are hydrochloride or carbonate salts.

16. Sevelamer or pharmaceutically acceptable salts thereof, preferably hydrochloride or carbonate salts, obtainable by means of the process of any one of the preceding claims.

17. Sevelamer or pharmaceutically acceptable salts thereof according to Claim 16, **characterized by** an angle of rest of between 25.0° and 27.0° and/or a true density of between 1.25 and 1.32 and/or a swelling index of between 7 and 9.

## Patentansprüche

1. Verfahren zur Herstellung von vernetzten Polyallylaminen oder pharmazeutisch akzeptablen Salzen davon, umfassend die folgenden Schritte:
a) Polymerisierung von Allylamin gemäß Formel (II) 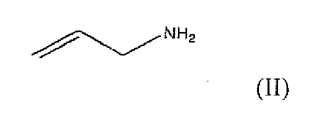 in Gegenwart eines Radikalstarters, in einer sauren Umgebung, in einem polaren Lösungsmittel oder einer Mischung aus polaren Lösungsmitteln, um ein Polyallylamin zu ergeben,
b) Vernetzen des in Schritt a) erhaltenen Polyallylamins in einer basischen Umgebung mit einem Vernetzer gemäß Formel (III) 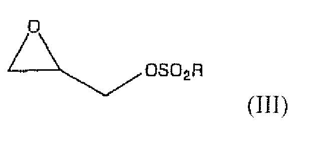 worin R für eine C₁-C₆ Alkylgruppe oder eine Arylgruppe, gegebenenfalls substituiert mit einer oder mehreren Alkylgruppen und/oder Elektronenziehenden Gruppen steht, in einem polaren Lösungsmittel oder einer Mischung aus polaren Lösungsmitteln, um ein Polymer zu ergeben;
c) Salzbildung des in Schritt b) erhaltenen Polymers, vorzugsweise mit einem pharmazeutisch akzeptablen Salz; und
d) gegebenenfalls Unwandlung des Polymersalzes in ein anderes pharmazeutisch akzeptables Salz.

2. Verfahren nach Anspruch 1, **dadurch gekennzeichnet, dass** der Radikalstarter ausgewählt ist aus nitrierten Verbindungen, vorzugsweise Azobisisobutyronitril, 2,2'-Azobis(2-amidinopropan)dihydrochlorid oder 2-2'Azobis[2-(2-imidazolin-2-yl)propan]dihydrochlorid, organischen Peroxiden, vorzugsweise Benzoylperoxid, Di-tert-amylperoxid, Dicumylperoxid, Isopropylbenzoperoxid, anorganische Peroxide, vorzugsweise Wasserstoffperoxid, Natriumperoxid, Kaliumperoxid, Verbindungen, die durch thermische oder Redox-Reaktionen aktiviert werden können, oder eine Kombination davon, besonders bevorzugt ist der Radikalstarter eine nitrierte Verbindung, insbesondere bevorzugt 2.2'-Azobis(2-amidinopropan)dihydrochlorid oder 2-2'Azobis[2-(2-imidazolin-2-yl)propan]dihydrochlorid.

3. Verfahren gemäß Anspruch 1, **dadurch gekennzeichnet, dass** das Molverhältnis von Allylamin zu Radikalstarter zwischen 20:1 und 50:1, vorzugsweise zwischen 20:1 und 35:1 liegt.

4. Verfahren nach Anspruch 1, **dadurch gekennzeichnet, dass** die Polymerisationsreaktion des Schrittes a) in Gegenwart einer anorganischen Säure durchgeführt wird, vorzugsweise Salzsäure, Bromwasserstoffsäure, Schwefelsäure, Salpetersäure oder Phosphorsäure, besonders bevorzugt Salzsäure.

5. Verfahren nach Anspruch 1, **dadurch gekennzeichnet, dass** das polare Lösungsmittel ausgewählt ist aus Wasser, einem Ester, vorzugsweise Ethylacetat oder Butylacetat, einem Nitril, vorzugsweise Acetonitril, einem Ether, vorzugsweise Tetrahydrofuran oder Dioxan, einem Amid, vorzugsweise Dimethylformamid oder Dimethylacetamid, einem Sulfoxid, vorzugsweise Dimethylsuifoxid oder Mischungen davon, besonders bevorzugt Wasser.

6. Verfahren nach Anspruch 1, **dadurch gekennzeichnet, dass** die Polymerisationstemperatur beim Schritt a) zwischen der Zersetzungstemperatur des Radikalstarter und der Rückflusstemperatur der Reaktionsmischung liegt, vorzugsweise zwischen 60 und 80°C.

7. Verfahren nach Anspruch 1, **dadurch gekennzeichnet, dass** die R-Gruppe des Vernetzers gemäß Formel (III) aus Schritt b) 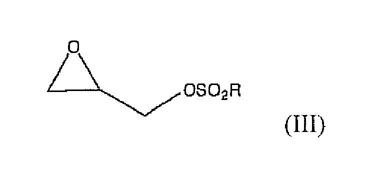 ausgewählt ist aus Methyl, Trifluormethyl, p-Tolyl und *m*-Nitrophenyl, vorzugsweise *m*-Nitrophenyl oder *p*-Tolyl.

8. Verfahren nach Anspruch 7, **dadurch gekennzeichnet, dass** der Vernetzer gemäß Formel (III) *m*-Nitrobenzolsulfonylglycidol oder Tosylglycidol ist.

9. Verfahren nach Anspruch 1, **dadurch gekennzeichnet, dass** das Vernetzungsmittel gemäß Formel (III) zu dem Polyamin aus Schritt a) in fester Form oder in Lösung zugesetzt wird, vorzugsweise in Lösung in einem polaren Lösungsmittel, besonders bevorzugt in Lösung in Wasser oder Ethylacetat.

10. Verfahren nach Anspruch 1, **dadurch gekennzeichnet, dass** die Konzentration des Vernetzers gemäß Formel (III) in Lösung zwischen 0,5 und 2 mol/l liegt, vorzugweise zwischen 1,2 und 1,8 mol/l.

11. Verfahren nach Anspruch 1, **dadurch gekennzeichnet, dass** die Vernetzungsreaktion des Schrittes b) durchgeführt wird in Gegenwart einer Base, vorzugsweise einer anorganischen Base, besonders bevorzugt Natrium oder Kaliumhydroxid, oder Natrium- oder Kaliumkarbonat, besonders bevorzugt Natriumhydroxid.

12. Verfahren nach Anspruch 1, **dadurch gekennzeichnet, dass** die Salzbildung des Schrittes c) durch Behandlung mit einer Säure stattfindet, vorzugsweise eine pharmazeutisch akzeptable Säure, insbesondere eine anorganische Säure, besonders bevorzugt Salzsäure.

13. Verfahren nach Anspruch 1, **dadurch gekennzeichnet dass** das pharmazeutisch akzeptable Salz des Schrittes d) Karbonat ist.

14. Verfahren nach Anspruch 1, **dadurch gekennzeichnet, dass** mindestens einer, besonders bevorzugt alle der Schritte a), b), c) und d) im Verfahren der vorliegenden Erfindung in einer "Eintopfreaktion" durchgeführt werden.

15. Verfahren gemäß einem der vorhergehenden Ansprüche, dadurch gekennzeichet, dass das vernetzte Polyallylamin Sevelanler ist, und dass die pharmazeutisch akzeptablen Salze davon Chlorwasserstoff oder Karbonatsalze sind.

16. Sevelamer oder pharmazeutisch akzeptable Salze davon, vorzugsweise Chlorwasserstoff- oder Karbonatsalze, erhältlich durch das Verfahren gemäß einem der vorhergehenden Ansprüche.

17. Sevelamer oder pharmazeutisch akzeptable Salze davon gemäß Anspruch 16, **gekennzeichnet durch** einen Ruhewinkel zwischen 25.0° und 27.0°, und/oder einer wahren Dichte zwischen 1,25 und 1,32, und/oder einem Quellindex zwischen 7 und 9.

## Revendications

1. Procédé de préparation de polyallylamines réticulées ou de sels pharmaceutiquement acceptables de celles-ci, qui comprend les étapes suivante :
a) la polymérisation d'une allylamine de formule (II) 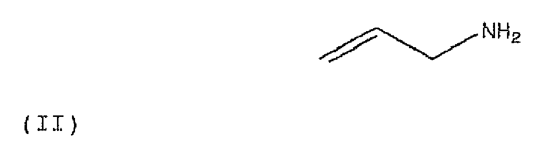 en présence d'un initiateur radicalaire, dans un environnement acide, dans un solvant polaire ou un mélange de solvants polaires pour donner une polyallylamine ;
b) la réticulation de la polyallylamine obtenue dans l'étape a) dans un environnement basique avec un agent de réticulation de formule (III) 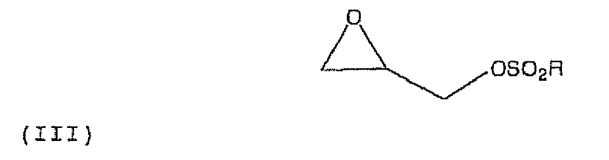 dans laquelle R représente un groupe allyle en C₁ à C₆, ou un groupe aryle éventuellement substitué par un ou plusieurs groupes alkyle et/ou groupes attracteurs d'électrons, dans un solvant polaire ou un mélange de solvants polaires pour donner un polymère ;
c) la salification du polymère obtenu dans l'étape b), de préférence avec un sel pharmaceutiquement acceptable ; et
d) la conversion éventuelle du polymère salifié en un autre sel pharmaceutiquement acceptable.

2. Procédé selon la revendication 1. **caractérisé en ce que** l'initiateur radicalaire est choisi parmi des composés nitrés, de préférence l'azobisisobutyronitrile, le dichlorhydrate de 2,2'-axobis(2-amidinopropane), ou le dichlorhydrate de 2,2'-azobis[2-(2-imidazolin-2-yl)propane], des peroxydes organiques, de préférence le peroxyde de benzoyle, le peroxyde de di-*tert*-amyle, le peroxyde de dicumyle, le peroxyde d'isopropylbenzene, des peroxydes inorganiques, de préférence le peroxyde d'hydrogène, le peroxyde de sodium, le peroxyde de potassium, des composés qui peuvent être activés par des réactions athermiques ou d'oxydoréduction, ou une combinaison de ceux-ci, plus particulièrement, l'initiateur radicalaire est un composé nitré, de manière même davantage préférée le dichlorhydrate de 2,2'-azobis(2-amidinopropane) ou le dichlorhydrate de 2,2'-azobis[2-(2-imidazolin-2-yl)propane].

3. Procédé selon la revendication 1, **caractérisé en ce que** le rapport molaire de l'allylamine sur l'initiateur radicalaire se situe entre 20/1 et 50/1, de préférence entre 20/1 et 35/1.

4. Procédé selon la revendication 1, **caractérisé en ce que** la réaction de polymérisation de l'étape a) est réalisée en présence d'un acide inorganique, de préférence l'acide chlorhydrique, l'acide bromhydrique, l'acide sulfurique, l'acide nitrique, ou l'acide phosphorique, de manière davantage préférée l'acide chlorlaydrique.

5. Procédé selon la revendication 1, **caractérisé en ce que** le solvant polaire est choisi parmi l'eau, un ester, de préférence l'acétate d'éthyle ou l'acétate de butyle, un nitrile, de préférence l'acétonitrile, un éther, de préférence le tétrahydrofurane ou le dioxane, un amide, de préférence le diméthylformamide ou le diméthylacétamide, un sulfoxyde, de préférence le sulfoxyde de diméthyle, ou des mélanges de ceux-ci, de manière davantage préférée l'eau.

6. Procédé selon la revendication 1, **caractérisé en ce que** la température de la polymérisation de l'étape a) se situe entre la température de décomposition de l'initiateur radicalaire et la température de reflux du mélange réactionnel, de préférence entre 60 et 80°C.

7. Procédé selon la revendication 1, **caractérisé en ce que** le groupe R de l'agent de réticulation de formule (III) de l'étape b) 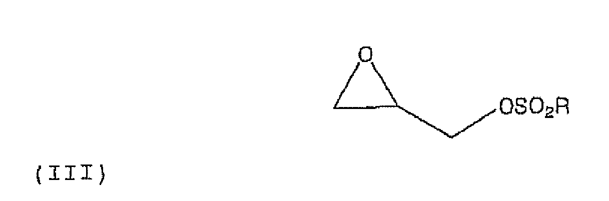 est choisi parmi les groupes méthyle, trifluorométhyle, *p*-tolyle, et *m*-nitro-phényle, de préférence *m*-nitro-phényle ou *p*-tolyle.

8. Procédé selon la revendication 7, **caractérisé en ce que** l'agent de réticulation de formole (III) est le *m*-nitrobenzènesulfonyl-glycidol ou le tosyl-glycidol.

9. Procédé selon la revendication 1, **caractérisé en ce que** l'agent de réticulation de formule (III) est ajouté à la polyamine de l'étape a) sous forme solide ou en solution, de préférence en solution dans un solvant polaire, de manière davantage préférée en solution dans de l'eau ou de l'acétate d'éthyle.

10. Procédé selon la revendication 1, **caractérisé en ce que** la concentration de l'agent de réticulation de formule (III) en solution se situe entre 0,5 et 2 mol/l, de préférence entre 1,2 et 1,8 mol/l.

11. Procédé selon la revendication 1, **caractérisé en ce que** la réaction de réticulation de l'étape b) est réalisée en présence d'une base, de préférence une base inorganique, de manière davantage préférée l'hydroxyde de sodium ou de potassium, ou le carbonate de sodium ou de potassium, de manière même davantage préférée l'hydroxyde de sodium.

12. Procédé selon la revendication 1, **caractérisé en ce que** la salification de l'étape c) prend place par traitement avec un acide, de préférence un acide pharmaceutiquement acceptable, de manière davantage préférée un acide inorganique, de manière même davantage préférée l'acide chlorhydrique.

13. Procédé selon la revendication 1, **caractérisé en ce que** le sel pharmaceutiquement acceptable de l'étape d) est un carbonate.

14. Procédé selon la revendication 1, **caractérisé en ce qu'**au moins l'une, et de manière même davantage préférée la totalité, des étapes a), b), c) et d) du procédé de la présente invention sont réalisées dans « un seul récipient » (« one pot »).

15. Procédé selon l'une quelconque des revendications précédentes, **caractérisé en ce que** la polyallylamine réticulée est le sevelamer et que les sels pharmaceutiquement acceptables de celui-ci sont les sels chlorhydrate ou carbonate.

16. Sevelamer ou des sels pharmaceutiquement acceptables de celui-ci, de préférence des sels chlorhydrate ou carbonate, pouvant être obtenus au moyen du procédé selon l'une quelconque des revendications précédentes.

17. Sevelamer ou des sels pharmaceutiquement acceptables de celui-ci selon la revendication 16, **caractérisés par** un angle de repos situé entre 25,0° et 27,0° et/ou une densité vraie située entre 1,25 et 1,32 et/ou un indice de gonflement situé entre 7 et 9.
